# EUROPEAN PATENT SPECIFICATION

(11) **EP 2 191 811 B1**
(45) Date of publication and mention of the grant of the patent: **06.11.2013**
(21) Application number: 08020674.1
(22) Date of filing: 28.11.2008
(51) Int. Cl.: A61K 8/04, A61K 8/34, A61K 8/41, A61Q 5/12

(54) **Conditioning composition for keratin fibres**
Zusammensetzung zum Konditionieren von Keratinfasern
Composition de conditionnement pour des fibres de kératine

(43) Date of publication of application: 02.06.2010
(73) Proprietor: Kao Germany GmbH, 64297 Darmstadt (DE)
(72) Inventor: Hoffmann, Martin, 64673 Zwingenberg (DE); Hermes, Frank, 64665 Alsbach-Hähnlein (DE)
(74) Representative: Grit, Mustafa

(56) References cited:
- EP-A- 1 174 111
- EP-A- 1 174 112
- EP-A- 1 382 324
- EP-A- 1 752 128
- WO-A-01/43718
- WO-A-2004/058199
- DE-U1-202005 009 617
- US-A1- 2005 136 011
- US-A1- 2006 034 792

## Description

Present invention relates to a conditioning composition for keratin fibres especially human hair in an aerosol foam form.

Aerosol foam conditioning compositions for keratin fibres especially hair have been known for many years. They comprise conditioning ingredients, surfactants, propellant and packed in a pressurised can. The product is delivered in form of foam which is then applied onto hair by hands. Such kinds of products are especially used in hair styling area and usually not rinsed off from hair after application. The foam delivered from the pressurized can is compact and very stable and does normally not liquidify during application onto hair and therefore usually longer application time is needed, and product is difficult to distribute.

There is a great need for developing foam conditioning composition delivered from a pressurized container which is delivered in the form of highly stable compact foam but liquidifies in a shorter period of time when worked into hair by hands which is furthermore easily be distributed into hair.

WO 01/43718 discloses conditioning hair compositions comprising cationic surfactants and further ingredients such as fatty alcohols, which are intended for use as mousse formulations with a propellant. The examples show such compositions comprising behenyl trimethyl ammonium chloride and behenyl alcohol.

The inventors of the present application have surprisingly found out that a conditioning composition according to claim 1 for keratin fibres especially hair comprises at least one fatty alcohol and at least one cationic surfactant wherein fatty alcohol and cationic surfactant has the same number of C atoms in their alkyl chains which is packed into an aerosol can together with a propellant and delivered in the form of a compact and viscous foam liquidifies when worked into hair by hands.

Accordingly, the first object of the present invention is a conditioning composition for keratin fibres, especially hair, according to claim 1, comprising at least one fatty alcohol, at least one cationic surfactant, and at least one propellant wherein fatty alcohol and cationic surfactant has the same number of C atoms in their alkyl chain.

Composition of the present invention conditions hair and improves hair properties in terms of combability, detangling, elasticity, shine, manageability, volume and body. The compositions are suitable for use as a rinse off composition, i.e. composition is rinsed off from hair after certain period of processing time after application onto hair and also as a leave in composition, i.e. the composition is applied onto hair and not rinsed off from hair.

Further object of the present invention is process for conditioning hair wherein a composition of the present invention is applied onto hair and after a processing time of 30 s to 45 min rinsed off from hair.

Still further object of the present invention is process for conditioning hair wherein a composition of the present invention is applied onto hair and hair is dried without rinsing off.

Another object of the present invention is the use of composition according to claim 1 comprising at least one fatty alcohol, at least one cationic surfactant, and at least one propellant wherein fatty alcohol and cationic surfactant has the same number of C atoms in their alkyl chain for conditioning hair, especially improving hair properties in terms of combability, detangling, elasticity, shine, manageability, volume and body.

Still another object of the present invention is use of composition according to claim 1 comprising at least one fatty alcohol, at least one cationic surfactant, and at least one propellant wherein fatty alcohol and cationic surfactant has the same number of C atoms in their alkyl chain for producing foam which liquidifies when worked into hair by hands.

Composition of the present invention comprise at least one fatty alcohol with a saturated or unsaturated, straight or branched alkyl chain with 12 to 24 C atoms in its alkyl chain. Preferably the alkyl chain length is 14 to 22 C atoms, more preferably 16 to 22 C atoms. Suitable non-limiting examples are lauryl alcohol, myristyl alcohol, stearyl alcohol, cetyl alcohol, behenyl alcohol, octyldodeqanol, arachidyl alcohol and oleyl alcohol. Preferred are myristyl alcohol, stearyl alcohol, cetyl alcohol, behenyl alcohol and octyldodecanol. More preferred are stearyl alcohol, cetyl alcohol, and behenyl alcohol. Most preferred fatty alcohol is behenyl alcohol.

In the preferred form of the present invention, mixture of fatty alcohols is generally excluded and therefore the compositions must comprise at least 90% by weight, preferably 95% by weight and most preferably solely one type of fatty alcohol. Concentration of fatty alcohol is in the range of 0.5 to 10%, preferably 0.5 to 7.5, more preferably 1 to 7.5 and most preferably 1 to 5% by weight calculated to total composition.

Composition of the present invention comprises at least one cationic or cationizable surfactant. Preferably the cationic or cationizable surfactant has only one long alkyl chain with 8 to 24 C atoms. More preferably the cationic or cationizable surfactant is selected from compounds according to the general structure 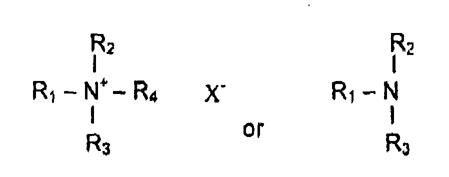 where R₁ is

R₅ CO NH (CH₂)ₙ

where R₅ is saturated or unsaturated, branched or straight alkyl chain with 7-23 C atoms and n has value of 1 - 4,
or

R₆ CO O (CH₂)ₙ

where R₆ is saturated or unsaturated, branched or straight alkyl chain with 7-23 C atoms and n has value of 1 - 4, and
R₂, R₃ and R₄ are same or different hydrogen or lower alkyl chain with 1 to 4 carbon atoms which may be substituted with one or two hydroxyl groups, and X is anion such as chloride, bromide, methosulfate, and behentrimonium chloride.

In the preferred embodiment of the present invention, compositions comprise at least one quaternary ammonium surfactant which is selected from the above given general structures. In more preferred embodiment of the present invention composition comprise quaternary ammonium surfactants without ester and amido bonds.
Typical examples of those ingredients are cetyltrimethyl ammonium chloride, steartrimonium chloride, behentrimonium chloride, stearamidopropyl trimonuim chloride, behenamidopropyltrimonium chloride. Most preferred quaternary ammonium compound is behentrimonium chloride,

Concentration of cationic surfactant is in the range of 0.1 to 5%, preferably 0.2 to 4%, more preferably 0.4 to 3% and most preferably 0.5 to 2.5% by weight calculated to total composition.

Compositions of the present invention comprise at least one propellant. Propellant is preferably alkane or a haloalkene with 2 to 4 C atoms such as propane, butane, isobutene and most preferably it is a mixture of propane and butane. It should be noted that with other known propellants such as dimethyl ether, carbondioxide, liquid nitrogen and air it may also function.

Concentration of at least one propellant or mixture of propellants is in the range of 1 to 20%, preferably 1 to 15%, more preferably 2 to 12.5% and most preferably 2 to 10% by weight calculated to total composition.

Composition of the present invention comprise at least one fatty acid alkyl ester of the general structure

R₇ C (O) O R₈

wherein R₇ is saturated or unsaturated, branched or straight alkyl chain with 7-23 C atoms and R₈ is saturated or unsaturated, branched or straight alkyl chain with 1 to 24 C atoms. Preferably, R₈ is 1 to 12, more preferably 1 to 8 and most preferably 1 to 4 C atoms.

Suitable examples are behenyl behenate, behenyl isostearte, butyl stearate, butyl oleate, butyl myristate,butyloctyl oleate,cetyl palmitate, cetyl myristate, cetyl oleate, cetyl caprylate, cetyl caprate, decyl oleate, decyl cocoate, decyl isostearate, ethylhexyl myristate, ethyl hexyl laurate, ethyl hexyl oleate, ethyl isostearte, ethyl laurate, ethyl linoleate, ethyl myristate, ethyl oleate, ethyl palmitate, ethylricinoleate, ethyl stearate, hexyl isostearet, hexyl laurate, hexyl myristate, hexyl stearate, hexyl decyl oleate, isobutyl laurate, isobutyl myristate, isobutyl palmitate, isobutyl stearate, isocetyl behenate, isobutyl laurate, isobutyl oleate, isobutyl stearate, isobutyl cocoate, isohexyl caprate, isopropyl palmitate, isopropyl stearate, isopropyl behenate, isopropyl laurate, isopropyl oleate, isopropyl ricinoleate, isopropyl palmitate,

Concentration of at least one fatty acid alkyl ester is in the range of 0.01 to 5%, preferably 0.05 to 4% and more preferably 0.1 to 3% by weight calculated to total composition.

Composition of the present invention may comprise at least one polyol at a concentration of 0.1 to 10% by weight calculated to total composition. Within the meaning of the present invention with the term polyol it is meant any compound comprising 2 or more hydroxyl group in its molecule. Suitable and preferred examples are glycerol, panthenol and polyethylene and/or propylene glycols.

Composition of the present invention may further comprise nonionic surfactants Examples are long-chain fatty acid mono- and dialkanolamides, such as coco fatty acid mono- or diethanolamide and myristic fatty acid mono or diethanolamide, stearic acid mono or diethanolamide. Further nonionic surfactants suited are alkyl polyglucosides with an alkyl group of 8 to 18 carbon atoms, and with 1 to 5 glucoside units. Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates. Further nonionic surfactants preferred in the dyeing compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 25, preferably about 10 and about 20. Fatty alcohol ethoxylates are the most preferred ones according to the present invention.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Nonionic surfactants are the most preferred ones. Concentration of nonionic surfactant is in the range of 0.1 to 10% by weight calculated to total composition.

The composition according to the present invention can contain organic solvent. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total composition.

Composition of the present invention can comprise pyrrolidone carboxylic acid esters. Some examples to the suitable pyrrolidon carboxylic acid ester are with the alkyl chain of methyl, ethyl, n-propyl, isopropyl, n-butyl, iso butyl, n-hexyl, n-octyl, n-nonyl, n-decyl, ethylhexyl, methylhexyl, capryl, lauryl myristyl, cetyl, octyldodecyl, isostearyl, stearyl, arachidyl, behenyl, oleyl, linoleyl, benzyl, phenoxyethyl. Preferred are n-octyl, n-nonyl, n-decyl, ethylhexyl, methylhexyl, capryl, lauryl myristyl, cetyl, octyldodecyl, stearyl, isostearyl, arachidyl, behenyl, oleyl and linoleyl. More preferred are lauryl myristyl, cetyl, octyldodecyl, stearyl, isostearyl, arachidyl, behenyl, oleyl, and linoleyl. Particularly preferred pyrrolidon carboxylic acid ester is octyldodecyl pyrrolidon carboxylic acid which is available under the trade name Protelan ODPC from Zschimmer & Schwarz.

Concentration of at least one pyrrolidon carboxylic acid ester in the compositions of the present invention is in the range of 0.01 to 5%, preferably 0.05 to 4%, more preferably 0.1 to 3% and most preferably 0.25 to 2% by weight calculated to total composition. It should be noted that compositions of the present invention can comprise more than one pyrrolidon carboxylic acid ester such as 2 or 3. The above mentioned concentrations refer to the total concentration of the pyrrolidon carboxylic acid esters.

Composition of the present invention comprises pyrrolidon carboxylic acid and/or its salts. In principal any pyrrolidon carboxylic acid salt is suitable within the meaning of the present invention. Suitable examples are aluminium, calcium, copper, magnesium, potassium, sodium and zinc salts of pyrrolidon carboxylic acid. Preferred are aluminium, calcium, magnesium, potassium and sodium salts and more preferred are sodium and potassium salts. The most preferred is sodium salt.

Concentration of pyrrolidon carboxylic acid and/or its salts is in the range of 0.01 to 10%, preferably 0.05 to 7.5%, more preferably 0.1 to 5% and most preferably 0.2 to 3% by weight calculated to the total composition Concentrations mentioned here refer to the total concentration of the pyrrolidon carboxylic acid and/or its salts present in the compositions.

Another preferred compound in the composition of present invention is ceramide type of compounds according to general formula 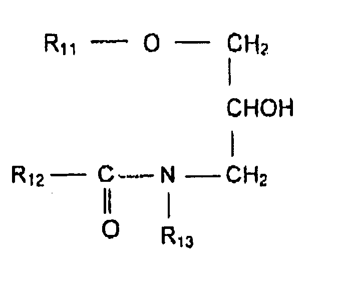 wherein R₁₁ and R₁₂ are independent from each other alkyl- or alkenyl group with 10 to 22 carbon atoms, R₁₃ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01 to 2%, preferably 0.01 to 1% by weight calculated to total composition.

Another preferred compound in the composition of present invention is ubichinone type of compounds according to general formula 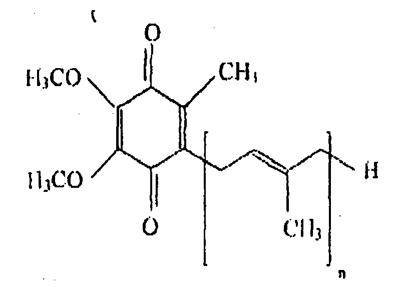 wherein n is a number from 1 to 10. Concentration of ubichinone can vary between 0.001 % and 1 % by weight, calculated to the total composition,

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethaxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebornane-2-one and the salts thereof and/or 3-(4'-methyl benzylidene)-DL-campher, polysilicone-15. The preferred amount of the UV-absorber ranges from about 0.01 % to 2.5%, more preferably from 0.05 % to 1 % by weight, calculated to the total composition.

According to the preferred embodiment of the present invention, the foam composition comprises at least one direct dye. Suitable direct dyes are anionic, cationic and non-ionic nitro dyes.

Suitable anionic direct dyes in aqueous composition are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27 and DC Yellow 10.

Suitable cationic dyes in aqueous composition are in principal those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31. The most preferred ones are Basic red 51, Basic Yellow 87 and Basic Orange 31 sold by CIBA.

Additionally, the aqueous compositions of the present invention may comprise neutral dyes (HC dyes), so called nitro dyes for shading purposes. Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Mellow No.11, HC Yellow No.12, HC Yellow No.13, HC Yellow Na.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

From the above disclosed direct dyes the preferred are cationic and nitro dyes and most preferred are cationic direct dyes for hair colouring puposes especially in rinse off application.

According to the invention, the third composition comprises one or more direct dye at a concentration of 0.01 to 2.5% by weight calculated to the total composition. The composition can also comprise mixture of several direct dyes i.e. an anionic, a cationic and/or a nonionic ones. In such a case the dyes may be mixed at any ratio with each other.

pH of the aqueous composition of the present invention varies between 2 and 7, preferably 2.5 - 6, more preferably 3 to 5.5, and most preferably 3 to 5. pH is adjusted to the required pH by using citric acid, malic acid, lactic acid, maleic acid, phosphoric acid,and glycolic acid, as basic compounds where necessary, monoethanolamine, triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

Compositions of the present invention can comprise additional hair conditioning compounds such as oils, cationic polymers, non-ionic substances. Oils as conditioners according to the present invention are selected from silicone oils either volatile or non-volatile, natural and synthetic oils. Among silicone oils those can be added to the compositions include dimethicone, dimethiconol, polydimethylsiloxane and DC fluid ranges from Dow Corning, cyclosiloxanes such as DC 245, arylated silicones phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and trimethyl pentaphenyl trisiloxane. Synthetic oils include mineral oil such as paraffin oil and petrolatum.

Natural oils suitable are such as olive oil, almond oil, avocado oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof.

Concentration of synthetic and/or natural oils mentioned above in the compositions of the present invention is in the range of 0.01 to 5% by weight calculated to total composition.

Composition of the present invention comprises cationic polymers as conditioning agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium. Typical examples of those Polyquaternium 4, Polyquaternium 6, Polyquaternium 7, Polyquaternium 10, Polyquaternium 11, Polyquaternium 18, Polyquaternium 22, Polyquaternium 24, Polyquaternium 28, Polyquaternium 30, Polyquaternium 37, Polyquaternium 36, Polyquaternium 46, Polyquaternium 67 and Polyquaternium 72.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640643.

Concentration of one or more cationic polymers is in the range of 0.1 to 2% by weight calculated to total composition.

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R} ". Extracts and the preparation thereof are also described in "Hagers Handbuch der pharmazeutischen Praxis", 4th Ed.

Viscosity of the emulsion compositions according to the present invention prior to confectioning in an aerosol can together with propellant is preferably below 2,000 mPa.s and more preferably 1,500 mPa.s and most preferably 1,000 mPa.s measured at 20°C with a Brookfield viscosimetre with an appropriate spindle and speed, preferably with Spindle 1 and at 5 rpm.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

The composition of the present invention can contain additional ingredients such as preservatives, chelating agents, fragrance and substances customarily used in cosmetic bleaching compositions of keratin fibres, especially hair.

Following examples are to illustrate the invention but not to limit.

### Example 1

| | % by weight |
|---|---|
| Behenyl alcohol | 3.0 |
| Behentrimonium chloride | 1.0 |
| Citric acid | q.s. to pH 4.5 |
| Preservative, fragrance | q.s. |
| Water | q.s. to 94 |
| Propane/butane 4.2 Bar | 6 |

The above composition was prepared by melting behenyl alcohol and behentrimonium chloride in water while stirring continuously at approximately 70°C and subsequently the mixture was cooled down to approximately 40°C and pH was adjusted with citric acid solution to 4.5 and preservative, fragrance and the reaming little portion of water was mixed and the mixture cooled down to ambient temperature and confectioned in an aerosol can with propane/butane mixture.

For comparative purposes the above composition was produced once with steartrimonium chloride as the cationic surfactant and the other time with stearyl alcohol with keeping the concentrations constant. The two comparative compositions were tested against the inventive composition and found out that in all case spreadability of the inventive composition was judged to be far better compared to both of the comparative compositions since the inventive composition liquidified easier than the other two comparatives.

In order to test the foam stability the following method was used:

A funnel with an outlet opening diameter of 4 cm and an outlet pipe length of 5 cm was filled with the foam at the upper reservoir and the foam was mixed with a paddle mixer with a diameter of 5 cm at a speed of 100 rpm and the time for filling the outlet pipe was measured. It was observed that the time for inventive composition was 3 min and the time for comparative compositions were 6 min independent from the type of alcohol and/or type of cationic surfactant. This shows clearly that the inventive composition produces foam which liquidifies much more quickly than the comparatives.

Similar results were obtained in terms of spreadability.

### Example 2

| | % by weight |
|---|---|
| Behenyl alcohol | 3.0 |
| Behentrimonium chloride | 1.0 |
| Isopropyl myristate | 1.0 |
| Citric acid | q.s. to pH 4.0 |
| Preservative, fragrance | q.s. |
| Water | q.s. to 94 |
| Propane/butane 4.2 Bar | 6 |

### Example 3

| | % by weight |
|---|---|
| Behenyl alcohol | 3.0 |
| Behentrimonium chloride | 2.0 |
| Isopropyl palmitate | 3.0 |
| Mineral oil | 1.0 |
| Dimethicone | 0.5 |
| Lactic acid | q.s. to pH 4.0 |
| Preservative, fragrance | q.s. |
| Water | q.s. to 94 |
| Propane/butane 4.2 Bar | 6 |

The above composition was produced in the same way as in example wherein oil or oily compounds are mixed together with fatty alcohol and cationic surfactant. The composition had a viscosity of 600 mPa.s, measured at 20°C with a Brookfield Viscosimetre Spindle 1 at 5 rpm, and was judged to have excellent spreading properties on hair. Additionally it improves hair combability, shine and makes hair exceptionally soft. The composition was tested in a monadic test design with female volunteers having approximately shoulder length hair. Amount of composition applied to whole head was approximately 10 g onto freshly shampooed and towel dried hair and processed for 5 min and rinsed off from hair.

In another monadic test the same composition was tested as a leave in composition. Volunteers having shoulder length hair was washed and towel dried and approximately 3 g of the foam was first rubbed in hands and applied onto hair and hair was further dried with a drier. It was observed that hair became excellently combable, had good shine and felt soft upon touching. Especially importantly it was reported that hair volume and body was increased.

The following compositions were produced in the same way as above and also tested in a monadic test design and judged to improve hair properties in combing, shine, elasticity, volume and body. The tests were carried out with a rinse off application and also in leave in application.

### Example 4

| | % by weight |
|---|---|
| Behenyl alcohol | 3.0 |
| Behentrimonium chloride | 1.0 |
| Isopropyl myristate | 1.0 |
| Benzophenone-3 | 0.2 |
| Glycerin | 3.0 |
| Citric acid | q.s. to pH 4.0 |
| Preservative, fragrance | q.s. |
| Water | q.s. to 94 |
| Propane/butane 4.2 Bar | 6 |

### Example 5

| | % by weight |
|---|---|
| Behenyl alcohol | 3.0 |
| Behentrimonium chloride | 1.0 |
| Isopropyl palmitate | 2.0 |
| Phenyltrimethicone | 2.0 |
| Benzophenone-3 | 0.2 |
| Panthenol | 1.0 |
| Lactic acid | q.s. to pH 4.0 |
| Preservative, fragrance | q.s. |
| Water | q.s. to 94 |
| Propane/butane 4.2 Bar | 6 |

### Example 6

| | % by weight |
|---|---|
| Behenyl alcohol | 3.0 |
| Behentrimonium chloride | 1.0 |
| Isopropyl palmitate | 2.0 |
| Trimethyl pentaphenyl trisiloxane | 2.0 |
| Polysilicone-15 | 0.2 |
| Panthenol | 1.0 |
| Lactic acid | q.s. to pH 4.0 |
| Preservative, fragrance | q.s. |
| Water | q.s. to 94 |
| Propane/butane 4.2 Bar | 6 |

### Example 7

| | % by weight |
|---|---|
| Behenyl alcohol | 5.0 |
| Behentrimonium chloride | 2.2 |
| Mineral oil | 1.0 |
| Trimethyl pentaphenyl trisiloxane | 2.0 |
| Ethylhexyl methoxy cinnamate | 0.2 |
| Panthenol | 1.0 |
| Basic red 51 | 0.1 |
| Lactic acid | q.s. to pH 4.0 |
| Preservative, fragrance | q.s. |
| Water | q.s. to 94 |
| Propane/butane 4.2 Bar | 6 |

The above composition additionally gave hair a red shimmery shine in a rinse off application.

## Claims

1. Conditioning composition for keratin fibres, especially human hair, **characterised in**
**that** it comprises at least one fatty alcohol, at least one cationic and cationizable surfactant selected from compounds of the general structure 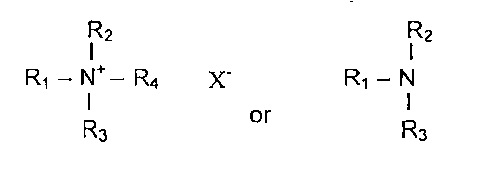 where R₁ is
R₅ CO NH (CH₂)ₙ
where R₅ is saturated or unsaturated, branched or straight alkyl chain with 7-23 C atoms and n has value of 1 - 4,
or
R₆COO(CH₂)ₙ
where R₆ is saturated or unsaturated, branched or straight alkyl chain with 7-23 C atoms and n has value of 1 - 4, and
R₂, R₃ and R₄ are same or different hydrogen or lower alkyl chain with 1 to 4 carbon atoms which may be substituted with one or two hydroxyl groups, and X is anion such as chloride, bromide, methosulfate, and behentrimonium chloride, at least one propellant and at least one fatty acid alkyl ester of the general structure
R₇C(O)OR₈
wherein R₇ is saturated or unsaturated, branched or straight alkyl chain with 7-23 C atoms and R₈ is saturated or unsaturated, branched or straight alkyl chain with 1 to 24 C atoms, preferably, R₈ is 1 to 12, more preferably 1 to 8 and most preferably 1 to 4 C atoms, and /or at least one natural oil, and/or at least one mineral oil wherein at least one fatty alcohol and at least one cationic and cationizable surfactant have the same number of C atoms in their alkyl chain.

2. Composition according to claim 1 **characterised in that** the composition comprise only one fatty alcohol and only one cationic and/or cationizable surfactant.

3. Composition according to any of the preceding claims **characterised in that** fatty alcohol is behenyl alcohol and cationic surfactant is behentrimonium chloride.

4. Composition according to any of the preceding claims **characterised in that** it comprises propane-butane mixture as propellant.

5. Composition according to any of the preceding claims **characterised in that** it comprises at least one polyol.

6. Composition according to any of the preceding claims **characterised in that** it comprises at least one additional surfactant selected from non-ionic and amphoteric ones, preferably non-ionic surfactant.

7. Composition according to any of the preceding claims **characterised in that** it comprises at least one organic solvent

8. Composition according to any of the preceding claims **characterised in that** it comprises pyrrolidone carboxylic acid and/or its salts and/or its esters.

9. Composition according to any of the preceding claims **characterised in that** it comprises at least one UV filter and/or at least one ubichinone of the general structu re 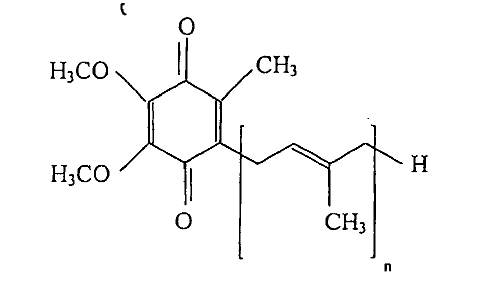 wherein n is a number from 1 to 10.

10. Use of a composition according to any of the preceding claims for conditioning hair, especially for improving hair properties in terms of combability, elasticity, shine, volume and body.

11. Use of composition according to claims 1 to 9 for producing foam which liquidifies when worked into hair by hands.

12. Process for conditioning hair **characterised in that** a composition according to claims 1 to 9 is applied onto hair and after proceeding time of 30 s to 45 min rinsed off from hair.

13. Process for conditioning hair **characterised in that** a composition according to claims 1 to 9 is applied onto hair and hair is dried, preferably with a hair drier without rinsing off.

## Patentansprüche

1. Konditionierende Zusammensetzung für Keratinfasern, speziell menschliche Haare, **dadurch gekennzeichnet, dass** sie mindestens einen Fettalkohol und mindestens ein kationisches und kationisierbares Tensid ausgewählt aus Verbindungen der generellen Struktur 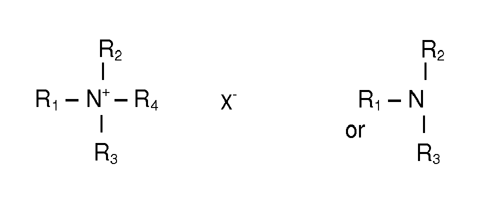 WO R₁ ist R₅ CO NH (CH₂)ₙ
wo R₅ eine gesättigte oder ungesättigte, verzweigte oder gerade Alkylkette mit 7 - 23 C-Atomen ist und n einen Wert von 1 - 4 hat, oder
R₆ CO O (CH₂)ₙ
wo R₆ eine gesättigte oder ungesättigte, verzweigte oder gerade Alkylkette mit 7 - 23 c- Atomen ist und n einen Wert von 1 - 4 hat, und
R₂, R₃ und R₄ gleich oder unterschiedlich Wasserstoff oder niedrige Alkylketten mit 1 bis 4 Kohlenstoffatomen sind die mit einer oder zwei Hydroxylgruppen substituiert sein können und X ein Anion wie Chlorid, Bromid, Methosulfat und Behentrimoniumchlorid, minderstens ein Treibgas und mindestens einen Fettsäure- Alkylester der allgemeinen Struktur
R₇ C (O) O R₈
worin R₇ eine gesättigte oder ungesättigte, verzweigte oder gerade Alkylkette mit 7 - 23 C-atomen ist und R₈ eine gesättigte oder ungesättigte, verzweigte oder gerade Alkylkette mit 1 bis 24 C- Atomen, vorzugsweise mit 1 bis 12, besonders bevorzugt 1 bis 8 und ganz besonders bevorzugt 1 bis 4 C- Atomen ist, und/oder ein natürliches Oel, und/oder mindestens ein Mineralöl enthält,
worin mindestens ein Fettalkohol und mindestens ein kationisches und kationisierbares Tensid die gleiche Anzahl von C- Atomen in ihrer Alkylkette hat.

2. Zusammensetzung nach Anspruch 1, **dadurch gekennzeichnet, dass** die Zusammensetzung nur einen Fettalkohol und nur ein kationisches und/oder kationisierbares Tensid enthält.

3. Zusammensetzung nach einem der vorhergehenden Ansprüche **dadurch gekennzeichnet, dass** Fettalkohol Behenylalkohol und das kationische Tensid Behentrimoniumchlorid ist.

4. Zusammensetzung nach einem der vorhergehenden Ansprüche, **dadurch gekennzeichnet, dass** sie Propan- Butanmischung als Treibgas enthält.

5. Zusammensetzung nach einem der vorhergehenden Ansprüche, **dadurch gekennzeichnet, dass** sie mindestens ein Polyol enthält.

6. Zusammensetzung nach einem der vorhergehenden Ansprüche, **dadurch gekennzeichnet, dass** sie mindestens ein zusätzliches Tensid, ausgewählt aus nicht ionischen und amphoteren, vorzugsweise ein nicht ionisches Tensid enthält.

7. Zusammensetzung nach einem der vorhergehenden Ansprüche, **dadurch gekennzeichnet, dass** sie mindestens ein organisches Lösungsmittel enthält.

8. Zusammensetzung nach einem der vorhergehenden Ansprüche, **dadurch gekennzeichnet, dass** sie Pyrrolidoncarboxylsäure und/oder ihre Salze und/oder ihre Ester enthält.

9. Zusammensetzung nach einem der vorhergehenden Ansprüche, **dadurch gekennzeichnet, dass** sie mindestens einen UV- Filter und oder mindestens ein Ubichinon mit der allgemeinen Struktur 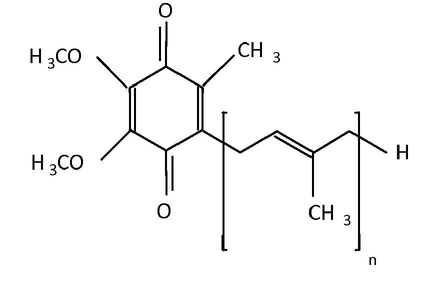 enthält,
worin n eine Zahl von 1 bis 10 ist.

10. Verwendung einer Zusammensetzung nach einem der vorhergehenden Ansprüche zur Konditionierung von Haaren, speziell zur Verbesserung der Haareigenschaften im Bezug auf Kämmfähigkeit, Elastizität, Glanz, Volumen und Fülle.

11. Verwendung einer Zusammensetzung nach den Ansprüchen 1 bis 9 zur Erstellung von Schaum der sich verflüssigt wenn er per Hand in das Haar eingearbeitet wird.

12. Verfahren zur Konditionierung von Haaren, **dadurch gekennzeichnet**, das seine Zusammensetzung nach den Ansprüchen 1 bis 9 auf das Haar aufgetragen wird und nach einer Einwirkzeit von 30 bis 45 Minuten aus dem Haar ausgespült wird.

13. Verfahren zur Konditionierung von Haaren, **dadurch gekennzeichnet, dass** eine Zusammensetzung nach einem der Ansprüche 1 bis 9 auf das Haar aufgetragen wird und das Haar getrocknet wird, vorzugsweise mit einem Haartrockner, ohne sie auszuspülen.

## Revendications

1. Composition de conditionnement pour les fibres kératiniques, en particulier pour les cheveux, **caractérisée par le fait qu'**elle comprend au moins un alcool gras, au moins un tensioactif cationique et cationisable choisi parmi les composés de formule générale : 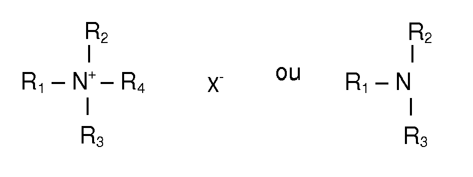 où R₁ est R₅ CO NH (CH₂)ₙ
où R₅ est une chaîne alkyle saturée ou insaturée, ramifiée ou linéaire avec 7-23 atomes de C et n a une valeur typique de 1 - 4, ou
R₆ CO O (CH₂)ₙ
où R₆ est une chaine alkyle saturée ou insaturée, ramifiée ou linéaire avec 7-23 atomes C et n a une valeur typique de 1 - 4 et
R₂, R₃ et R₄, identiques ou différents, sont choisis parmi les atomes d'hydrogènes ou des chaînes alkyles inférieures avec 1 à 4 atomes de carbone qui peuvent être substitués avec un ou deux groupes hydroxyles, et X est un anion tel que le chlorure, bromure, méthosulfate et le chlorure de behentrimonium, au moins un propulseur et au moins un ester d'alkyle d'acide gras de formule générale
R₇ C (O) O R₈
où R₇ est une chaîne alkyle saturée ou insaturée, ramifiée ou linéaire avec 7 à 23 atomes de carbone et R₈ est une chaîne alkyle saturée ou insaturée, ramifiée ou linéaire avec 1 à 24 atomes de carbone. De préférence R₈ est formé de 1 à 12, plus particulièrement de 1 à 8 et encore plus particulièrement de 1 à 4 atomes de carbone, et/ou d'au moins une huile naturelle, et/ou d'au moins une huile minérale,
où au moins un acide gras et au moins un surfactant cationique et cationisable ont le même nombre d'atomes de carbone dans leur chaîne alkyle.

2. Composition selon la revendication 1, **caractérisée par le fait que** la composition ne comprend qu'un alcool gras et qu'un tensioactif cationique et/ou cationisable.

3. Composition, selon l'une quelconque des revendications précédentes, **caractérisée par le fait que** l'alcool gras est le docosanol et que le tensioactif cationique est le chlorure de behentrimonium.

4. Composition, selon l'une quelconque des revendications précédentes, **caractérisée par le fait qu'**elle comprend un mélange propane-butane comme propulseur.

5. Composition, selon l'une quelconque des revendications précédentes, **caractérisée par le fait qu'**elle comprend au moins un polyol.

6. Composition, selon l'une quelconque des revendications précédentes, **caractérisée par le fait qu'**elle comprend au moins un tensioactif supplémentaire choisi parmi les tensioactifs non-ioniques et amphotères, de préférence les tensioactifs non-ioniques.

7. Composition, selon l'une quelconque des revendications précédentes, **caractérisée par le fait qu'**elle comprend au moins un solvant organique

8. Composition, selon l'une quelconque des revendications précédentes, **caractérisée par le fait qu'**elle comprend de l'acide pyrrolidone carboxylique et/ou ses sels et/ou ses esters.

9. Composition, selon l'une quelconque des revendications précédentes, **caractérisée par le fait qu'**elle comprend au moins un filtre UV et/ou au moins une ubiquinone de formule générale 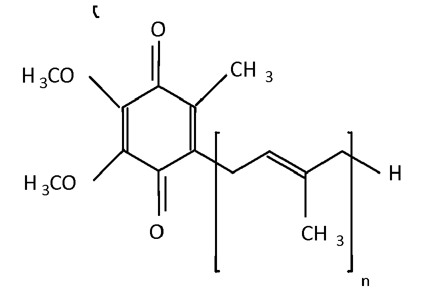 où n est un nombre entre 1 et 10

10. Utilisation d'une composition selon l'une quelconque des revendications précédentes pour le conditionnement des cheveux, en particulier pour améliorer les propriétés des cheveux en termes de démêlage, élasticité, brillance, volume et corps.

11. Utilisation d'une composition selon les revendications 1 à 9 pour produire une mousse qui se liquéfie lorsqu'elle est travaillée dans la chevelure avec les mains.

12. Procédé de conditionnement des cheveux **caractérisé par le fait qu'**une composition, selon les revendications 1 à 9, est appliquée sur la chevelure et après un temps de pose de 30 secondes à 45 minutes est rincée.

13. Procédé de conditionnement des cheveux **caractérisé par le fait qu'**une composition, selon les revendications 1 à 9, est appliquée sur la chevelure et la chevelure est séchée, sans rinçage, de préférence avec un séchoir.
